(19) Europäisches Patentamt
European Patent Office
Office européen des brevets

(11) **EP 2 575 924 B1**

(12) **EUROPÄISCHE PATENTSCHRIFT**

(45) Veröffentlichungstag und Bekanntmachung des Hinweises auf die Patenterteilung:
**05.08.2015 Patentblatt 2015/32**

(21) Anmeldenummer: **11728775.5**

(22) Anmeldetag: **03.06.2011**

(51) Int Cl.:
***A61M 1/30*** *(2006.01)*

(86) Internationale Anmeldenummer:
**PCT/EP2011/002736**

(87) Internationale Veröffentlichungsnummer:
**WO 2011/151075 (08.12.2011 Gazette 2011/49)**

(54) **VORRICHTUNG ZUR BLUTBEHANDLUNG IM EINNADEL-BETRIEB**

DEVICE FOR BLOOD TREATMENT IN SINGLE-NEEDLE MODE

DISPOSITIF DE TRAITEMENT DU SANG À AIGUILLE UNIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.06.2010 DE 102010022776**

(43) Veröffentlichungstag der Anmeldung:
**10.04.2013 Patentblatt 2013/15**

(73) Patentinhaber: **Fresenius Medical Care Deutschland GmbH**
**61352 Bad Homburg v.d.H. (DE)**

(72) Erfinder:
• **BOCKLET, Christoph**
  **97708 Bad Bocklet (DE)**

• **SCHÄFER, Ingo**
  **65760 Eschborn (DE)**
• **THYS, Martin**
  **97508 Grettstadt (DE)**
• **NOACK, Joachim**
  **97616 Bad Neustadt (DE)**
• **MÜLLER, Ralf**
  **61348 Bad Homburg (DE)**

(74) Vertreter: **Oppermann, Frank**
**OANDO Oppermann & Oppermann LLP**
**Washingtonstrasse 75**
**65189 Wiesbaden (DE)**

(56) Entgegenhaltungen:
**EP-A2- 0 405 094    DE-A1-102007 026 009**

**Beschreibung**

Die Erfindung bezieht sich auf eine Vorrichtung zur Blutbehandlung für den Einnadel-Betrieb, die über einen extrakorporalen Blutkreislauf verfügt, der eine zu dem Einlass einer Blutbehandlungseinheit führende Blutzuführleitung und eine von dem Auslass der Blutbehandlungseinheit abgehende Blutrückführleitung aufweist, wobei Blutzuführ- und -rückführleitung zu einer gemeinsamen Nadel (Kanüle) führen bzw. von einer gemeinsamen Nadel abgehen.

Blutbehandlungsvorrichtungen mit einer Blutbehandlungseinheit, die von dem Blut eines Patienten durchströmt wird, sind allgemein bekannt. Zu diesen zählen beispielsweise die bekannten Hämodialyse-, Hämofiltrations- oder Hämodiafiltrationsvorrichtungen. Die bekannten Blutbehandlungsvorrichtungen können im Einnadel- oder Zweinadel-Betrieb betrieben werden.

Bei der Zweinadel-Technik wird das Blut über eine erste Nadel von einem Blutgefäß des Patienten abgezogen, in die Blutbehandlungseinheit der Blutbehandlungsvorrichtung geleitet und über eine zweite Nadel in ein Blutgefäß des Patienten zurückgeführt. Für die Entnahme und Rückführung des Blutes finden auswechselbare Schlauchsysteme mit einer Blutzuführ- und -rückführleitung Verwendung, an denen die beiden Nadeln angeschlossen sind. Diese wegzuwerfenden Schlauchsysteme werden auch als Disposable bezeichnet.

Bei der Einnadel-Technik erfolgt die Entnahme und Rückführung des Blutes über eine einzige Nadel. Das dem Patienten entnommene Blut wird während einer arteriellen Phase in einem Reservoir gespeichert, um dann in einer venösen Phase aus dem Speicher in den Blutkreislauf des Patienten durch dieselbe Nadel zurückgeführt zu werden.

Die EP 0 405 094 A2 beschreibt eine Vorrichtung zur Blutbehandlung im Einnadel-Betrieb, die über einen Plasmaseparator verfügt. Der Einnadel-Betrieb umfasst zwei Schritte. In dem ersten Schritt wird das Blut des Patienten in einem Blutsammelbeutel gesammelt. In dem zweiten Schritt werden das gesammelte Blut aus dem Blutsammelbeutel in den Plasmaseparator überführt, das Blutplasma aus dem Plasmaseparator in einen Plasmasammelbeutel überführt und die verbleibenden Blutkomponenten zu dem Patienten zurückgeführt. Der extrakorporale Blutkreislauf umfasst eine Blutzuführleitung und eine Blutrückführleitung, die zu einer gemeinsamen Nadel führen. Der Blutsammelbeutel befindet sich in einer Aufnahmeeinheit, in der der Beutel einem Über- bzw. Unterdruck ausgesetzt wird. Der Über- oder Unterdruck wird in der Aufnahmeeinheit für den Blutsammelbeutel mit einem Kompressor hergestellt, der über Leitungen, die Ventile enthalten, mit der Aufnahmeeinheit in Verbindung steht. Das Funktionsprinzip der EP 0 405 094 A2 liegt darin, den Blutsammelbeutel in der Aufnahmeeinheit zu komprimieren bzw. expandieren, ohne dass der Blutsammelbeutel mit den Mitteln zum Zuführen von Gas in unmittelbarer Strömungsverbindung steht.

Eine Blutbehandlungsvorrichtung für den Einnadel-Betrieb ist auch aus der DE 10 2007 026 009 A1 bekannt. Die bekannte Blutbehandlungsvorrichtung verfügt über einen extrakorporalen Blutkreislauf, der eine zu dem Einlass einer Blutbehandlungseinheit führende Blutzuführleitung und eine von dem Auslass der Blutbehandlungseinheit abgehende Blutrückführleitung aufweist. Dieser extrakorporale Blutkreislauf mit der Blutbehandlungseinheit braucht nicht Teil der Blutbehandlungsvorrichtung zu sein, sondern kann ein zur einmaligen Verwendung bestimmtes Disposable sein, das für die Blutbehandlung in die Behandlungseinheit eingelegt wird.

Darüber hinaus weist die Blutbehandlungsvorrichtung Mittel zum Sammeln von Blut und Mittel zum Speichern von Gas, insbesondere Luft auf. Bei den Mitteln zum Sammeln von Blut und zum Speichern von Gas handelt es sich jeweils um ein Reservoir, das ein abgeschlossenes Volumen bildet, beispielsweise um eine Blut- bzw. Luftkammer mit einem vorgegebenen Volumen.

Die Mittel zum Sammeln von Blut stehen mit den Mitteln zum Speichern von Gas in Verbindung, so dass in der arteriellen Phase beim Befüllen der Mittel zum Sammeln von Blut aus den Mitteln zum Sammeln von Blut verdrängte Luft in die Mittel zum Speichern von Gas und in der venösen Phase Luft aus den Mitteln zum Speichern von Gas in die Mittel zum Sammeln von Blut gelangen kann, wodurch das zuvor in der arteriellen Phase gesammelte Blut aus den Mitteln zum Sammeln von Blut verdrängt wird. Dadurch wird erreicht, dass in der arteriellen Phase dem Patienten Blut entnommen und in der venösen Phase das mit der Blutbehandlungseinheit behandelte Blut dem Patienten wieder zurückgeführt wird.

Die Verbindung zwischen den Mitteln zum Sammeln von Blut und den Mitteln zum Speichern von Gas weist einen ersten und zweiten Verbindungspfad auf. Der erste Verbindungspfad verbindet die Mittel zum Sammeln von Blut und zum Speichern von Gas derart, dass in der arteriellen Phase aus den Mitteln zum Sammeln von Blut verdrängte Luft zu den Mitteln zum Speichern von Gas überführt wird, wobei in dem ersten Verbindungspfad Mittel zum Unterbrechen der Verbindung vorgesehen sind. Der zweite Verbindungspfad verbindet die Mittel zum Sammeln von Blut und Speichern von Gas derart, dass in der venösen Phase in den Mitteln zum Speichern von Gas gespeicherte Luft zu den Mitteln zum Sammeln von Blut überführt wird. Der zweite Verbindungspfad enthält Mittel zum Verdichten von Gas, so dass die in den Mitteln zum Speichern von Gas gespeicherte Luft in die Mittel zum Sammeln von Blut überführt werden kann. Mit den Mitteln zum Verdichten der Luft kann in den Mitteln zum Sammeln von Blut ein vorgegebener Druck sowohl während der arteriellen als auch der venösen Phase aufgebaut werden. Die Mittel zum Verdichten von Gas können beispielsweise als ein konventioneller Kompressor ausgebildet sein.

Die Mittel zum Sammeln von Blut und Speichern von Gas bilden zusammen mit dem ersten und zweiten Verbindungspfad ein abgeschlossenes Volumen, in das kein Gas eintreten bzw. aus dem kein Gas austreten kann. Nur zum Zwecke der Initialisierung des Systems wird das abgeschlossene Volumen be-/entlüftet.

Für einen störungsfreien und sicheren Betrieb der Blutbehandlungsvorrichtung im Einnadel-Betrieb muss gewährleistet sein, dass unter keinen Umständen Luft in den Patienten infundiert werden kann.

Eine Luftinfusion in den Patienten soll bei der bekannten Blutbehandlungsvorrichtung dadurch ausgeschlossen werden, dass in den Mitteln zum Speichern von Gas ein Druck eingestellt wird, der in keinem Betriebsfall größer als der Druck ist, der sich in den Mitteln zum Sammeln von Blut einstellt. Dadurch soll erreicht werden, dass Luft nur mit den Mitteln zum Verdichten von Gas von den Mitteln zum Speichern von Gas in die Mittel zum Sammeln von Blut gelangen kann. Bei einem Ausfall der Mittel zum Verdichten von Gas, beispielsweise einer Leckage des Kompressors, soll hingegen keine Luft in die Mittel zum Sammeln von Blut gelangen können. Dadurch soll ausgeschlossen werden, dass die Mittel zum Sammeln von Blut unbeabsichtigt leer laufen, wodurch Luft in den Patienten gelangen könnte.

Bei der bekannten Blutbehandlungsvorrichtung erfolgt vor der Einleitung der arteriellen und venösen Phasen eine Initialisierung des Systems. Um die Bedingung, dass bei einem Ausfall des Kompressors nicht Luft aus den Mitteln zum Speichern von Gas in die Mittel zum Sammeln von Blut gelangen kann, bei niedrigem Rückgabedruck und großem Schlagvolumen erfüllen zu können, wird das System zur Initialisierung der aufeinander folgenden arteriellen und venösen Phasen zum Zeitpunkt der Umschaltung von der arteriellen auf die venöse Phase, d.h. zu dem Zeitpunkt des oberen Umschaltpunktes, bei dem die Mittel zum Sammeln von Blut befüllt sind, auf einen vorgegebenen Druck, vorzugsweise den Umgebungsdruck, entspannt. Der Druck in den Mitteln zum Speichern von Gas liegt somit in den aufeinander folgenden arteriellen und venösen Phasen immer unterhalb des vorgegebenen Drucks, insbesondere des Umgebungsdrucks, auf den das abgeschlossene System entspannt worden ist.

Als nachteilig erweist sich, dass vor der Einleitung der arteriellen und venösen Phasen die Mittel zum Sammeln von Blut solange mit Blut befüllt werden müssen, bis der Füllstand den oberen Umschaltpunkt erreicht hat. Darüber hinaus ist zuvor die Einstellung eines bestimmten Blutpegels in den Mitteln zum Sammeln von Blut erforderlich.

Der Erfindung liegt die Aufgabe zu Grunde, die Sicherheit der Blutbehandlung im Einnadel-Betrieb zu erhöhen und/oder die Initialisierung der aufeinander folgenden arteriellen und venösen Phasen zu vereinfachen.

Die Lösung dieser <u>Aufgabe</u> erfolgt erfindungsgemäß mit den Merkmalen des <u>Patentanspruchs 1.</u> Vorteilhafte Ausführungsformen der Erfindung sind Gegenstand der Unteransprüche.

Die erfindungsgemäße Vorrichtung <u>beruht</u> auf der Berechnung eines Drucks, der in den Mitteln zum Speichern von Gas vor der Einleitung der arteriellen und venösen Phasen eingestellt wird. Dieser maximale Druck ist derart bemessen, dass unter keinen Umständen Luft in den Patienten gelangen kann. Bei der Berechnung des Drucks werden zwei Randbedingungen berücksichtigt. Einerseits sollte der Druck klein genug sein, um sicherzustellen, dass sowohl eine Luftinfusion in den Patienten ausgeschlossen ist als auch bei Gegendrücken, die kleiner als der Umgebungsdruck sind, noch eine Regelung des Blutflusses in den Patienten sichergestellt ist. Andererseits sollte der Druck groß genug sein, um für die Verdrängung des Bluts aus den Mitteln zum Sammeln von Blut in den Mitteln zum Speichern von Gas eine ausreichend große Luftmasse zur Verfügung stellen zu können.

Da der in den Mitteln zum Speichern von Gas einzustellende Druck bei dem erfindungsgemäßen Verfahren und der erfindungsgemäßen Vorrichtung berechnet wird, ist es nicht erforderlich, die Drücke in den Mitteln zum Sammeln von Blut und den Mitteln zum Speichern von Gas bei niedrigstem und höchstem Füllstand zu messen. Darüber hinaus kann die Initialisierung zur Einleitung der arteriellen und venösen Phasen unabhängig von dem momentanen Flüssigkeitspegel jederzeit eingeleitet werden. Es ist weder erforderlich, den Flüssigkeitspegel auf den niedrigsten und höchsten Füllstand einzustellen, noch sind Mittel zur Bestimmung des Flüssigkeitspegels erforderlich.

Der zuvor berechnete (Unter-)Druck kann vor der Einleitung der arteriellen und venösen Phasen einfach dadurch eingestellt werden, dass Gas aus den Mitteln zum Speichern von Gas, vorzugsweise in die Atmosphäre, abgeführt wird. In einzelnen Fällen, beispielsweise bei einem besonders hohen venösen Gegendruck, bei besonderen Patientenlagen oder bei besonderen geodätischen Höhenlagen kann es aber auch sinnvoll sein, wenn in den Mitteln zum Speichern von Gas ein Überdruck eingestellt wird. Zur Einstellung eines Überdrucks wird Gas den Mitteln zum Sammeln von Blut zugeführt.

Zur Berechnung des in den Mitteln zum Speichern von Gas einzustellenden Drucks wird vorzugsweise das in den Mitteln zum Sammeln von Blut enthaltene Volumen an Gas ermittelt und auf der Grundlage des in den Mitteln zum Sammeln von Blut enthaltenen Gasvolumens wird dann der in den Mitteln zum Speichern von Gas einzustellende Druck bestimmt. Anstelle des in den Mitteln zum Sammeln von Blut enthaltenen Gasvolumens kann auch das in den Mitteln zum Sammeln von Blut enthaltene Volumen an Blut bestimmt werden, um auf der Grundlage des in den Mitteln zum Sammeln von Blut enthaltenen Blutvolumens den einzustellenden Druck zu berechnen. Da das in den Mitteln zum Sammeln von Blut eingeschlossene Volumen bekannt ist, kann aus dem Blutvolumen das Gasvolumen oder umgekehrt jeweils berechnet werden.

Zur Ermittlung des in den Mitteln zum Sammeln von Blut enthaltenen Volumens an Gas wird vorzugweise ein vorgegebener Druckunterschied zwischen den Mitteln zum Sammeln von Blut und den Mitteln zum Speichern von Gas aufgebaut. Dieser Druckunterschied kann in der Höhe grundsätzlich beliebig sein. Hierzu wird Gas aus den Mitteln zum Speichern von Gas in die Mittel zum Sammeln von Blut überführt, wobei der absolute Druck in den Mitteln zum Speichern von Gas und der absolute Druck in den Mitteln zum Sammeln von Blut vor und nach dem Aufbau des Druckunterschieds bestimmt wird. Dabei ist unerheblich, wie der Druck in den Mitteln zum Speichern von Gas und der absolute Druck in den Mitteln zum Sammeln von Blut bestimmt werden. Beispielsweise kann der Druck mit Drucksensoren gemessen werden, die jeweils innerhalb des eingeschlossenen Volumens der Mittel zum Speichern von Gas und Mittel zum Sammeln von Blut angeordnet sind. Die Drücke können grundsätzlich aber auch in mit den Mitteln zum Sammeln von Blut und Speichern von Gas in Verbindung stehenden Leitungen gemessen werden, wenn sich Leitungsdruck und Kammerdruck angeglichen haben. Zur Bestimmung der absoluten Drücke in den Mitteln zum Speichern von Gas und Sammeln von Blut können absolute Drücke oder relative Drücke gemessen werden.

Eine bevorzugte Ausführungsform sieht zur Bestimmung der absoluten Drücke bei Messung relativer Drücke die Messung des Umgebungsdrucks vor. In den Mitteln zum Speichern von Gas und den Mitteln zum Sammeln von Blut wird jeweils der relative Druck gemessen. Die Werte des absoluten Drucks werden dann aus den Werten des relativen Drucks und des Umgebungsdrucks berechnet. Dies hat den Vorteil, dass zur Druckmessung in den Kammern relative Drucksensoren verwendet werden können, die zwar im Allgemeinen einen kleineren Messbereich, aber innerhalb des kleineren Messbereichs eine höhere Genauigkeit als absolute Drucksensoren haben. Darüber hinaus stehen relative Drucksensoren kostengünstig zur Verfügung.

Vor dem Überführen von Gas aus den Mitteln zum Speichern von Gas in die Mittel zum Sammeln von Blut wird in dem abgeschlossenen Volumen der Mittel zum Sammeln von Blut und in dem abgeschlossenen Volumen der Mittel zum Speichern von Gas ein Druck eingestellt wird, der dem Umgebungsdruck entspricht. Damit wird ein definierter Ausgangszustand für den Aufbau des Druckunterschieds hergestellt. Es ist aber auch möglich, den Druck in den Mitteln zum Sammeln von Blut zum Aufbau eines Druckunterschieds ausgehend von einem anderen Druck als den Umgebungsdruck zu erhöhen.

Zur Einstellung des Umgebungsdrucks wird vorzugsweise eine Verbindung zwischen den Mitteln zum Speichern von Gas und den Mitteln zum Sammeln von Blut hergestellt und die Mittel zum Speichern von Gas und/oder Mittel zum Sammeln von Blut be-/entlüftet. Für die Be-Entlüftung sind geeignete Mittel zum Be-/Entlüften vorgesehen. Da beide Mittel in Strömungsverbindung stehen, genügt es, wenn die Be- bzw. Entlüftung nur auf einer Seite stattfindet.

Bei einer bevorzugten Ausführungsform der erfindungsgemäßen Blutbehandlungsvorrichtung weisen die Mittel zur Herstellung einer Verbindung zwischen den Mitteln zum Sammeln von Blut und den Mitteln zum Speichern von Blut zwei Verbindungspfade auf. Der eine Verbindungspfad umfasst eine Leitung zum Fördern von Gas, die die Mittel zum Speichern von Gas mit den Mitteln zum Sammeln von Blut verbindet. Die Mittel zum Verdichten von Gas, beispielsweise ein Kompressor, sind in dieser Leitung angeordnet. Der andere Verbindungspfad umfasst eine die Mittel zum Verdichten von Gas umgehende Bypass-Leitung, in der ein Bypass-Ventil angeordnet ist. Wenn die Mittel zum Verdichten von Gas nicht betrieben werden, ist bei geschlossenem Bypass-Ventil das abgeschlossene Volumen der Mittel zum Sammeln von Blut von dem abgeschlossenen Volumen der Mittel zum Speichern von Gas getrennt. Wenn das Bypass-Ventil hingegen geöffnet ist, bilden beide Volumina ein gemeinsames abgeschlossenes Volumen. Für die Bestimmung des in den Mitteln zum Sammeln von Blut einzustellen Drucks ist es aber unerheblich, wie die Mittel zum Sammeln von Blut und die Mittel zum Speichern von Luft miteinander kommunizieren. Auch ist es unerheblich, wie Gas aus den Mitteln zum Sammeln von Blut in die Mittel zum Speichern von Luft oder umgekehrt überführt wird. Allein entscheidend ist, dass ein abgeschlossenes Gesamtvolumen zur Verfügung steht, dass nur zur Einleitung der Initialisierung be-/entlüftet wird.

Vorzugsweise sind die Mittel zum Sammeln von Blut als Behältnis mit einem vorgegebenen Volumen ausgebildet, das einen Einlass und einen Auslass aufweist und in der Blutrückführleitung des extrakorporalen Kreislaufs angeordnet ist. Auch die Mittel zum Speichern von Gas sind vorzugsweise als ein Behältnis mit einem vorgegebenen Volumen ausgebildet.

Die Mittel zum Sammeln von Blut sind vorzugsweise integraler Bestandteil einer Disposable-Kassette, insbesondere eine integrierte Single-Needle-Kammer in einer Disposable-Blutkassette für die Hämodialyse, wie sie in den Patentanmeldungen DE 10 2009 024 468 und PCT/EP2010/002488 beschrieben ist. Zumindest Teile der zu den bzw. von den Mitteln zum Sammeln von Blut zu- bzw. abführenden Blutleitungen sind vorzugsweise integraler Bestandteil der Blutkassette.

Behältnisse können Beutel aus einem flexiblen Material sein. Vorzugsweise sind die Behältnisse Folienbeutel, die zur einmaligen Verwendung bestimmt sind (Disposable). Wenn die Behältnisse als Disposable ausgebildet sind, muss aber durch eine geeignete Aufnahmeeinheit, in die sich die Folienbeutel einlegen lassen, sichergestellt sein, dass die Beutel positiven und negativen Gegendrücken standhalten, ohne zu kollabieren bzw. sich auszudehnen.

Das Blut im extrakorporalen Kreislauf wird vorzugsweise mit einer Blutpumpe gefördert, die in der Blutzuführleitung angeordnet ist.

Im Folgenden wird ein Ausführungsbeispiel der Erfindung unter Bezugnahme auf die Zeichnungen näher erläutert.

Es zeigen:

Fig. 1    ein Ausführungsbeispiel der erfindungsgemäßen Blutbehandlungsvorrichtung für den Einnadel-Betrieb in stark vereinfachter schematischer Darstellung,

Fig. 2    den Verlauf des Füllstandes und des Drucks während der Initialisierung und des Betriebs der erfindungsgemäßen Blutbehandlungsvorrichtung und

Fig. 3    den Druckverlauf während der aufeinander folgenden arteriellen und venösen Phasen.

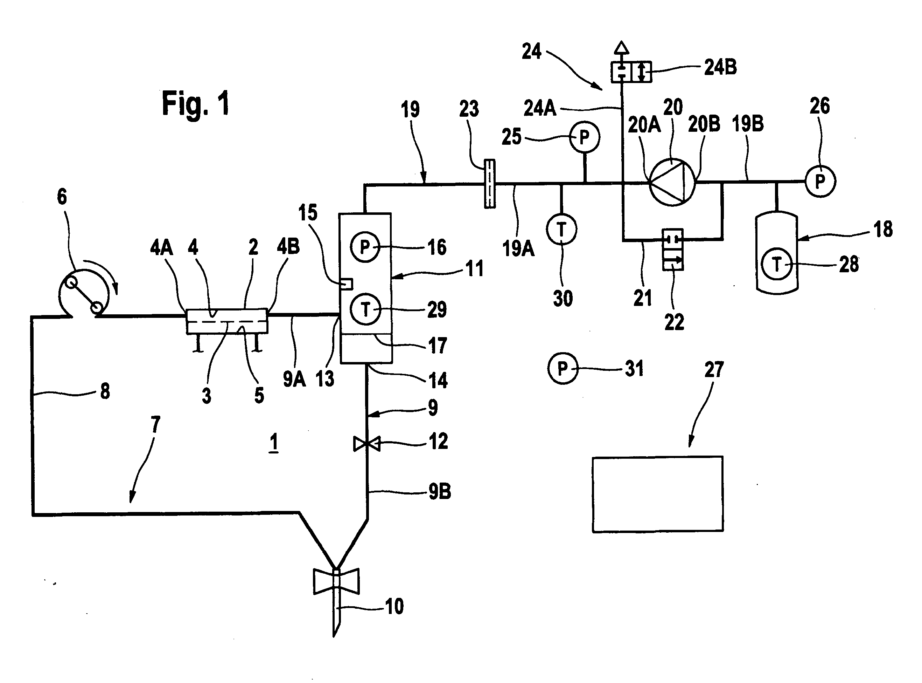

Fig. 1 zeigt die wesentlichen Komponenten einer Blutbehandlungsvorrichtung, insbesondere einer Dialysevorrichtung für den Einnadel-Betrieb in schematischer Darstellung, die in der DE 10 2007 026 009 A1 bis auf die erfindungsgemäße Initialiserung zur Einleitung der arteriellen und venösen Phasen im Einzelnen beschrieben ist.

Die Dialysevorrichtung verfügt während der Blutbehandlung über einen extrakorporalen Blutkreislauf 1, der eine als Disposable ausgebildete Blutbehandlungseinheit 2, beispielsweise einen Dialysator, umfasst. Der Dialysator 2 ist durch eine semipermeable Membran 3 in eine Blutkammer 4 und eine Dialysierflüssigkeitskammer 5 unterteilt.

Im extrakorporalen Blutkreislauf wird das Blut mittels einer Blutpumpe 6 gefördert, die Teil der Dialysevorrichtung ist. Der Dialysierflüssigkeitskreislauf ist in Figur 1 nicht dargestellt.

In die Dialysevorrichtung wird ein Schlauchset 7 eingelegt, das nach der Behandlung verworfen wird. Das Disposable 7 weist eine zu dem Einlass 4A der Blutkammer 4 des Dialysators 2 führende Blutzuführleitung 8, die in die Rollenpumpe 6 der Dialysevorrichtung eingelegt ist, und eine von dem Auslass 4B der Blutkammer abgehende Blutrückführleitung 9 auf. Blutzuführ- und -rückführleitung 8, 9 sind an einer gemeinsamen Kanüle 10 (Nadel) angeschlossen.

In der Blutrückführleitung 9 des Disposable sind Mittel 11 zum Sammeln von Blut angeordnet, die als ein Behälter mit einem vorgegebenen Volumen ausgebildet sind. Nachfolgend werden die Mittel zum Sammeln von Blut als Blutspeicher 11 bezeichnet. Stromab des Blutspeichers 11 sind an der Blutrückführleitung 9 Mittel 12 zum Unterbrechen der Blutrückführleitung, beispielsweise eine venöse Schlauchklemme angeordnet.

Der Blutspeicher 11 weist einen Einlass 13 auf, zu dem ein erster Abschnitt 9A der Blutrückführleitung 9 führt, und weist einen Auslass 14 auf, von dem ein zweiter Abschnitt 9B der Blutrückführleitung 9 abgeht. Zur Detektion eines bestimmten Füllstandes im Blutspeicher 11 kann die Dialysevorrichtung einen Füllstandgeber 15 aufweisen, der detektiert, wenn der Füllstand in dem Behälter einen vorgegebenen Wert erreicht. Darüber hinaus ist ein Druckgeber 16 vorgesehen, der den Druck im Blutspeicher 11 misst.

Wenn der Blutspeicher 11 mit Blut befüllt ist, verbleibt oberhalb des Flüssigkeitsspiegels 17 ein bestimmtes Luftvolumen in dem Blutspeicher. Der Blutspeicher 11 steht in Strömungsverbindung mit Mitteln 18 zum Speichern von Gas, insbesondere Luft, die als ein Behälter mit einem abgeschlossenen Volumen ausgebildet sind. Nachfolgend werden die Mittel 18 zum Speichern von Gas als Luftspeicher bezeichnet.

Damit Blutspeicher 11 und Luftspeicher 18 miteinander kommunizieren können, geht von der Oberseite des Blutspeichers 11 eine Leitung 19 ab, die zu dem Luftspeicher 18 führt. In der Leitung 19 sind Mittel 20 zum Verdichten von Gas angeordnet, die beispielsweise als konventioneller Kompressor ausgebildet sein können. Solange der Kompressor nicht betrieben wird, unterbricht der Kompressor die Strömungsverbindung zwischen Blutspeicher und Luftspeicher. Beim Betrieb des Kompressors wird hingegen in dem Luftspeicher befindliche Luft in den Blutspeicher überführt. Da die Luft komprimiert wird, baut sich in dem Blutspeicher ein vorgegebener Druck auf.

Die Leitung 19 weist zwei Leitungsabschnitte 19A, 19B auf, von denen der eine Leitungsabschnitt 19A den Blutspeicher 11 mit dem druckseitigen Anschluss 20A des Kompressors 20 und der andere Leitungsabschnitt 19B den saugseitigen Anschluss 20B des Kompressors 20 mit dem Luftspeicher 18 verbindet. Diese Leitungsabschnitte 19A, 19B bilden einen Verbindungspfad zum Überführen von Gas aus dem Luftspeicher in den Blutspeicher.

Um bei nicht im Betrieb befindlichem Kompressor 20 Luft aus dem Blutspeicher 11 in den Luftspeicher 18 überführen zu können, ist eine Bypassleitung 21 vorgesehen, die von dem ersten Leitungsabschnitt 19A der Leitung 19 abgeht und zu dem zweiten Leitungsabschnitt 19B der Leitung 19 führt. In die Bypassleitung 21 ist ein Bypassventil 22 geschaltet. Zusammen mit den entsprechenden Leitungsabschnitten der Leitung 19 bildet die Bypassleitung 21 einen Verbindungspfad zum Fördern von Gas aus dem Blutspeicher in den Luftspeicher.

Um zu verhindern, dass Flüssigkeit aus dem Blutspeicher 11 in den Luftspeicher 18 gelangen kann, ist in dem ersten Leitungsabschnitt 19A der Leitung 19 ein Filter 23 angeordnet, der eine hydrophobe, d.h. für Luft durchlässige, aber für Flüssigkeit undurchlässige Membran enthält. Da der Blutspeicher nur bis zu einem maximalen Füllstand befüllt wird, kann aber ohnehin nur im Falle einer Störung Flüssigkeit in die Leitung 19 gelangen.

Zum Be- / Entlüften des abgeschlossenen Volumens, das den Blutspeicher 11 und den Luftspeicher 18 sowie die Leitung 19 und die Leitung 21 umfasst, sind Mittel 24 zum Be- / Entlüften vorgesehen, die eine beispielsweise an den ersten Leitungsabschnitt 19A der Leitung 19 angeschlossene Be- / Entlüftungsleitung 24A mit einem Be- / Entlüftungsventil 24B aufweisen. Die Be- / Entlüftungsleitung 24A kann grundsätzlich von jeder Stelle des zu be- oder entlüftenden Volumens abgehen. Insbesondere sollte die Be- /Entlüftung im maschinenseitigen Teil erfolgen.

Zusätzlich zu dem Druckgeber 16 zum Messen des Drucks in dem Blutspeicher 11 ist ein Druckgeber 25 zum Messen des Drucks in dem ersten Leitungsabschnitt 19A der Leitung 19 zwischen dem Filter 23 und dem Kompressor 20 und ein weiterer Druckgeber 26 zum Messen des Drucks im Luftspeicher 18 vorgesehen.

Die Druckgeber 16, 25 und 26 sind bei dem vorliegenden Ausführungsbeispiel Drucksensoren zum Messen relativer Drücke. Zur Messung des Umgebungsdrucks ist ein weiterer Druckgeber 31 vorgesehen.

Darüber hinaus ist ein Temperatursensor 28 zum Messen der Temperatur der im Luftspeicher 18 befindlichen Luft, ein Temperatursensor 29 zum Messen der Temperatur der im Blutspeicher 11 befindlichen Luft und ein Temperatursensor 30 zum Messen der Temperatur der in dem ersten Leitungsabschnitt 19A der Leitung 19 befindlichen Luft vorgesehen.

Die Dialysevorrichtung verfügt über eine zentrale Steuer- und Recheneinheit 27, die über nicht dargestellte elektrische Leitungen mit der Blutpumpe 6, der venösen Schlauchklemme 12, dem Bypassventil 22, dem Be- / Entlüftungsventil 24B, dem Kompressor 20 und den Temperatursensoren 28, 29, 30 sowie den Druckgebern 16, 25, 26 zur Messung relativer Drücke und des Druckgebers 31 zum Messen des Umgebungsdrucks verbunden ist. Die zentrale Steuer- und Recheneinheit 27 steht wiederum mit einer nicht dargestellten Eingabeeinheit in Verbindung, die eine Schnittstelle zwischen dem Benutzer und der Maschine schafft.

Die Steuer- und Recheneinheit 27 berechnet die Werte der absoluten Drücke aus den mit den Druckgebern 16, 25 und 26 gemessenen Werten der relativen Drücke und dem mit dem Druckgeber 30 gemessenen Umgebungsdruck.

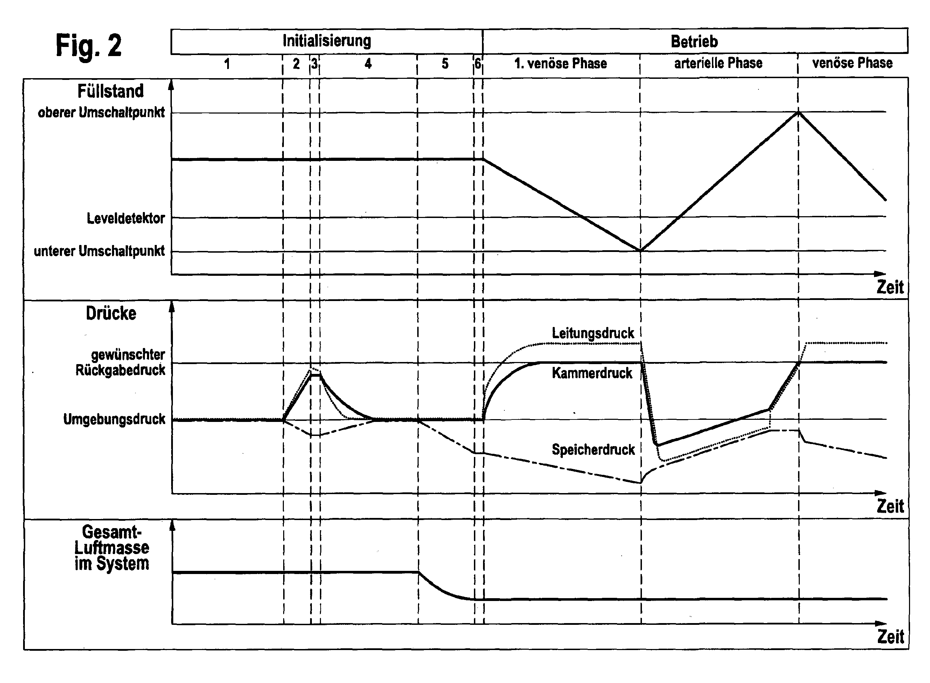

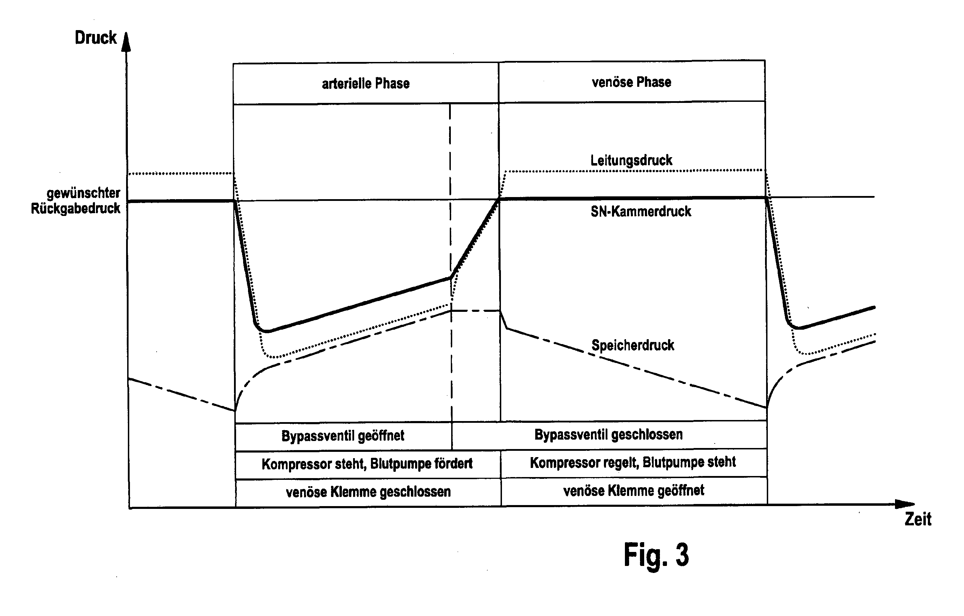

Nachfolgend wird der Betrieb der Dialysevorrichtung unter Bezugnahme auf die Figuren 2 und 3 im Einzelnen beschrieben. Die zentrale Steuer- und Recheneinheit 27 steuert die Dialysemaschine wie folgt.

Fig. 2 zeigt den Füllstand in dem Blutspeicher 11 als Funktion der Zeit während der einzelnen Phasen 1 bis 6 der Initialisierung. Darüber hinaus zeigt Fig. 2 in den einzelnen Initialisierungsschritten 1 bis 6 den Verlauf des Drucks im Blutspeicher 11, der als Kammerdruck $p_{Blutspeicher}$ bezeichnet wird, des Drucks in dem Luftspeicher 18, der als Speicherdruck $p_{Luftspeicher}$ bezeichnet wird, und des Drucks in dem ersten Leitungsabschnitt 19A der Leitung 19, der als Leitungsdruck $p_{Leitung}$ bezeichnet wird. Des Weiteren zeigt Fig. 2 den zeitlichen Verlauf der im Blutspeicher 11 und im Luftspeicher 18 sowie den entsprechenden Abschnitten der Leitungen 19, 22 eingeschlossenen Gesamt-Luftmasse in den einzelnen Initialisierungsschritten 1 bis 6.

Zu Beginn der eigentlichen Dialysebehandlung wird die erfindungsgemäße Initialisierung des Systems mit den nachfolgend beschriebenen Verfahrensschritten durchgeführt. Die Initialisierung kann grundsätzlich jederzeit während des Betriebs der Blutbehandlungsvorrichtung erfolgen, wobei ein bestimmtes Niveau des Bluts im Blutspeicher nicht erforderlich ist.

Unabhängig davon, ob es sich um die erste Initialisierung während der Blutbehandlung handelt, werden zu Beginn der Initialisierung von der Steuer- und Recheneinheit 27 das Bypassventil 22 sowie das Be-/Entlüftungsventil 24B geöffnet, um sowohl den Kammerdruck $p_{Blutspeicher}$ im Blutspeicher 11 als auch den Speicherdruck $p_{Luftspeicher}$ im Luftspeicher 18 auf Umgebungsdruck zu entspannen.

Daraufhin werden in einem nicht dargestellten Speicher der Steuer- und Recheneinheit 27 der sich zum Zeitpunkt $t_0$ nach der Be-/Entlüftung einstellende Kammerdruck $p_{Blutspeicher}$ im Blutspeicher 11 und der Speicherdruck $p_{Luftspeicher}$ im Luftspeicher 18 für die spätere Berechnung des im Blutspeicher 11 einzustellenden Drucks bestimmt. Der relative Kammerdruck $p_{Blutspeicher}$ kann mit dem Druckgeber 16 oder mit dem Druckgeber 25 gemessen werden, da davon auszugehen ist, dass sich sowohl in dem Blutspeicher 11 als auch in der mit dem Blutspeicher in Verbindung stehenden Leitung 19 der gleiche Druck, d.h. der Umgebungsdruck, eingestellt hat. Der relative Speicherdruck $p_{Luftspeicher}$ wird mit dem Druckgeber 26 gemessen. Die Steuer- und Recheneinheit 27 berechnet aus dem relativen Druckwerten und dem gemessenen Umgebungsdruck die absoluten Werte für den Kammerdruck $p_{Blutspeicher}$ und Speicherdruck $p_{Luftspeicher}$. Zur Vereinfachung können aber auch für den Kammerdruck $p_{Blutspeicher}$ im Blutspeicher 11 und den Speicherdruck $p_{Luftspeicher}$ im Luftspeicher 18 zum Zeitpunkt to der gemessene Umgebungsdruck angenommen werden (Schritt 1).

Danach werden das Bypassventil 22 sowie das Be-/Entlüftungsventil 24B von der Steuer- und Recheneinheit 27 geschlossen und der Kompressor 20 so lange in Betrieb gesetzt, bis der Kammerdruck $p_{Blutspeicher}$ einen vorgegebenen Wert erreicht hat (Schritt 2).

Anschließend wird nach einer Angleichung von dem Kammerdruck $p_{Blutspeicher}$ und dem Leitungsdruck $p_{Leitung}$ in der Leitung 19 der Kammerdruck $p_{Blutspeicher}$ im Blutspeicher 11 bzw. der Leitungsdruck $p_{Leitung}$ in der Leitung 19 und der Speicherdruck $p_{Luftspeicher}$ im Luftspeicher 18 zum Zeitpunkt $t_1$ bestimmt.

Nach der Druckmessung wird das Luftvolumen und somit der Flüssigkeitspegel im Blutspeicher 11 bestimmt (Schritt 3). Das Luftvolumen $V_{BlutspeicherLuft}$ im Blutspeicher 11 wird mit der Steuer- und Recheneinheit 27 nach der folgenden Gleichung (1) berechnet:

$$V_{BlutspeicherLuft} = -\frac{(p_{Luftspeicher\_t0} - p_{Luftspeicher\_t1})}{(p_{Blutspeicher\_t0} - p_{Blutspeicher\_t1\_})} \bullet V_{Luftspeicher} - V_{Leitung}$$

Gleichung (1)

$V_{BlutspeicherLuft}$ :   Berechnetes Luftvolumen im Blutspeicher

$V_{Luftspeicher}$ :   Bekanntes Volumen des Luftspeichers

$V_{Leitung}$ :   Bekanntes Volumen der Leitung

$p_{Luftspeicher\_t0}$ :   Speicherdruck vor Volumenverschiebung zum Zeitpunkt $t_0$

$p_{Luftspeicher\_t1}$ :   Speicherdruck nach Volumenverschiebung zum Zeitpunkt $t_1$

$p_{Blutspeicher\_t0}$ :   Kammerdruck vor Volumenverschiebung zum Zeitpunkt $t_0$

$p_{Blutspeicher\_t1}$ :   Kammerdruck nach Volumenverschiebung zum Zeitpunkt $t_1$

Nachdem das Luftvolumen $V_{BlutspeicherLuft}$ im Blutspeicher 11 bekannt ist, berechnet die Steuer- und Recheneinheit 27 nach der folgenden Gleichung (2) den maximal zulässigen Speicherdruck $p_{LuftspeicherMax}$ im Luftspeicher 18 zu dem ermittelten Kammerluftvolumen. Der maximal zulässige Speicherdruck $p_{LuftspeicherMax}$, der im Luftspeicher 18 eingestellt wird, soll beim Anfahren des oberen Umschaltpunktes im Blutspeicher 11 nicht zu einem Überdruck im Blutspeicher führen:

Gleichung (2)

$$p_{LuftspeicherMax} = (\hat{m}_{MaxGesamt} - \hat{m}_{Leitung} - \hat{m}_{Blutspeicher}) \cdot \frac{T_{Luftspeicher}}{V_{Luftspeicher}}$$

$$\hat{m}_{MaxGesamt} = \left(\frac{V_{Luftspeicher}}{T_{Luftspeicher}} + \frac{V_{Leitung}}{T_{Leitung}} + \frac{V_{Blutspeicher} - V_{MaxOT}}{T_{Blutspeicher}}\right) \cdot p_{Abs}$$

$$\hat{m}_{Leitung} = \frac{p_{Abs} \cdot V_{Leitung}}{T_{Leitung}}$$

$$\hat{m}_{Blutspeicher} = \frac{p_{Abs} \cdot V_{BlutspeicherLuft}}{T_{Blutspeicher}}$$

$p_{LuftspeicherMax}$ :   Einzustellender Druck im Luftspeicher (Speicherdruck)

$V_{MaxOT}$ :   Volumen im Blutspeicher bei maximalem Blutpegel im Betrieb

Die zur Berechnung des maximal zulässigen Speicherdrucks $p_{LuftspeicherMax}$ erforderlichen Werte für die Temperaturen in dem Blutspeicher $T_{Blutspeicher}$, dem Luftspeicher $T_{Luftspeicher}$ und der Leitung $T_{Leitung}$ erhält die Steuer- und Recheneinheit 27 von den entsprechenden Temperatursensoren 28, 29 und 30.

Bei dem vorliegenden Ausführungsbeispiel wird zur Vereinfachung der Berechnung bei den Gasgleichungen die Luftfeuchtigkeit nicht berücksichtigt. Auch finden die Parameter des Kompressors 20 keine Berücksichtigung. Um noch genauere Werte für den maximal zulässigen Speicherdrucks $p_{LuftspeicherMax}$ zu bestimmen, können auch die Luftfeuchtigkeit und/oder die Parameter des Kompressors noch Berücksichtigung finden. Hierzu sind entsprechende Luftfeuchtigkeits-Sensoren vorzusehen.

Das Bypassventil 22 sowie das Be-/Entlüftungsventil 24B werden nun geöffnet, um sowohl den Kammerdruck $p_{Blutspeicher}$ als auch den Speicherdruck $p_{Luftspeicher}$ auf Umgebungsdruck zu entspannen (Schritt 4). Damit wird ein definierter Ausgangszustand für die nachfolgende Anpassung der Luftmasse im System eingestellt. Anschließend wird das Bypassventil 22 geschlossen, wobei das Be-/Entlüftungsventil 24B geöffnet bleibt, und der Kompressor 20 wird so lange in Betrieb gesetzt, bis sich im Luftspeicher 18 ein Speicherdruck $p_{SpeicherMax}$ eingestellt hat, infolge dessen es in der späteren Behandlung beim Anfahren des oberen Umschaltpunktes nicht zu einem Überdruck im Blutspeicher kommt (Schritt 5). Dabei fördert der Kompressor 20 zur Reduzierung der Luftmasse im System über das Be-/Entlüftungsventil 24B Luft in die Umgebung.

Die Berechnung des maximal zulässigen Speicherdrucks $p_{LuftspeicherMax}$ wird anhand des vorliegenden Ausführungsbeispiels mit konkreten Zahlenwerten nochmals erläutert:

| | |
|---|---|
| Umgebungsdruck: | 750 mmHg |
| Aktueller Füllstand im Blutspeicher: | 35 ml |
| Maximaler Blutpegel im Blutspeicher: | 50 ml |

Bestimmung des Luftvolumens im Blutspeicher gemäß Gleichung 1:

$$V_{BlutspeicherLuft} = -\frac{(750mmHg - 700mmHg)}{(750mmHg - 1083mmHg)} \bullet 308ml - 7ml = 45ml$$

Bestimmung des einzustellenden Drucks in den Mitteln zum Speichern von Gas gemäß Gleichung 2:

$$p_{LuftspeicherMax} = (\hat{m}_{MaxGesamt} - \hat{m}_{Leitung} - \hat{m}_{Blutspeicher}) \cdot \frac{309K}{308ml} = 699mmHg$$

darin sind die Hilfsgrößen $\hat{m}_{MaxGesamt}$, $\hat{m}_{Leitung}$ und $\hat{m}_{Blutspeicher}$ für die Massen:

$$\hat{m}_{MaxGesamt} = \left(\frac{308ml}{309K} + \frac{7ml}{309K} + \frac{80ml - 50ml}{309K}\right) \cdot 750mmHg$$

$$\hat{m}_{Leitung} = \frac{750mmHg \cdot 7ml}{309K}$$

$$\hat{m}_{Blutspeicher} = \frac{750mmHg \cdot 45ml}{309K}$$

Im Luftspeicher 18 wird mittels des Kompressors 20 ein (Unter-)druck von 699 mmHg eingestellt. Damit ist sichergestellt, dass nach der Initialisierung während der arteriellen und venösen Phasen bei maximalem Blutpegel im Blutspeicher 11 sich im Blutspeicher kein über dem Umgebungsdruck von 750 mmHg liegender Überdruck einstellt, der zu einer Gefährdung des Patienten führen könnte.

Als letzter Schritt wird die im System enthaltene Luftmasse berechnet (Schritt 6). Dies wird später noch im Einzelnen beschrieben. Das Be-/Entlüftungsventil 24B bleibt während der gesamten Blutbehandlung geschlossen, wenn nicht eine erneute Initialisierung, beispielsweise nach der Detektion einer Luftleckage, erforderlich ist. Damit ist die Initialisierung abgeschlossen, und die Blutbehandlung startet mit der ersten venösen Phase bzw. wird mit der venösen Phase fortgesetzt.

Die Luftmasse bleibt nach dem Schließen des Be-/und Entlüftungsventils im System konstant. Mit dem Kompressor 20 können durch Luftmassenverschiebungen verschiedene Arbeitspunkte eingestellt werden. Die Summe der Luftmassen muss aber immer der Luftmasse zum Zeitpunkt der Be-/Entlüftung entsprechen. Ist dies nicht der Fall, hat einer oder mehrere der Druckgeber einen Fehler. Mit den Fehlern, die sich in den verschiedenen Arbeitspunkten ergeben, kann durch mathematische und statistische Methoden auf die Fehler der einzelne Sensoren geschlossen werden, so dass eine Korrektur des Fehlers möglich wird. Die Analyse der einzelnen Sensoren auf Fehler über die Analyse der Luftmassen bei verschiedenen Arbeitspunkten ist unabhängig von der vorgeschriebenen Initialisierung sowohl vor der Initialisierung (Regelfall) als auch nach der Initialisierung oder auch während der Behandlung möglich.

Nach der oben beschriebenen Initialsierung des Systems schließen sich die venösen und arteriellen Phasen an.

In der ersten venösen Phase wird der Kompressor 20 bei geschlossenem Bypassventil 22 betrieben, wobei die venöse Schlauchklemme 12 geöffnet ist und die Blutpumpe 6 still steht. Während des Betriebs des Kompressors wird Luft aus dem Luftspeicher 18 komprimiert und dem Blutspeicher 11 zugeführt. Dadurch nehmen Kammer- und Leitungsdruck zu, während der Speicherdruck abnimmt. Gleichzeitig nimmt der Füllstand im Blutspeicher kontinuierlich ab, bis das Niveau des unteren Umschaltpunkts erreicht ist. Dabei ist entscheidend, dass der Speicherdruck unterhalb des Kammerdrucks und folglich auch unterhalb des Leitungsdrucks liegt, um im Falle eines Fehlers einen Gaseintrag von dem Luftspeicher in den Blutspeicher zu verhindern. Zusätzlich wird angestrebt, dass der Speicherdruck sogar unterhalb des Umgebungsdrucks liegt.

Daraufhin beginnt die arterielle Phase, in der der Blutspeicher wieder mit dem Patienten entnommenen Blut befüllt wird, woraufhin sich wieder die venöse Phase anschließt, in der das Blut aus dem Blutspeicher wieder dem Patienten zugeführt wird.

Der Luftspeicher ist so groß dimensioniert, dass auch am Ende der venösen Phase genügend Luft im System vorhanden ist, um den gewünschten Rückgabedruck in dem Blutspeicher aufrechterhalten zu können. Um mit der gleichen Initialisierung sämtliche Betriebspunkte mit einem Rückgabedruck von 0 bis 500 mmHg relativ bei einem Schlagvolumen bis 60 ml einstellen zu können, wird in der Praxis ein Luftspeicher mit einem Speichervolumen von ca. 300 ml benötigt.

Der zeitliche Verlauf des Kammer- und Speicherdrucks sowie des Leitungsdrucks während der eigentlichen arteriellen und venösen Phasen nach der Initialisierung des Systems ist in Fig. 3 dargestellt, die einen Ausschnitt von Fig. 2 zeigt.

Während der gesamten arteriellen Phase wird die Blutpumpe 6 betrieben, wobei der Kompressor 20 stillsteht. Die venöse Schlauchklemme 12 bleibt während der gesamten arteriellen Phase geschlossen.

Zu Beginn der arteriellen Phase öffnet die Steuer- und Recheneinheit 27 das Bypassventil 22, so dass die aus dem Blutspeicher 11 verdrängte Luft über die Bypassleitung 21 in den Luftspeicher 18 gelangt. Folglich steigt der Speicherdruck an, während der Kammer- und Leitungsdruck zunächst absinkt, um dann ebenfalls gleichsam mit dem Speicherdruck anzusteigen. Die im Blutspeicher und in dem zugehörigen Leitungsvolumen enthaltene Luftmasse nimmt damit kontinuierlich ab.

Sobald die im Blutspeicher und dem Leitungsvolumen enthaltene Luftmasse einen vorgegebenen Betrag erreicht hat, der sich aus dem gewünschten Schlagvolumen und dem gewünschten Rückgabedruck ergibt, schließt die Steuer- und Recheneinheit das Bypassventil. Folglich entstehen zwei getrennte Luftvolumina, d.h. das Luftvolumen des Blutspeichers mit den zugehörigen Leitungsabschnitten und das Volumen des Luftspeichers mit den zugehörigen Leitungsabschnitten. Daraufhin wird die Blutpumpe bei geschlossenem Bypassventil betrieben, so dass der Speicherdruck konstant bleibt, während die Luft in dem Blutspeicher und dem zugehörigen Leitungsvolumen so lange verdichtet wird, bis bei Erreichen des gewünschten Schlagvolumens auch der gewünschte Rückgabedruck erreicht ist. Figur 3 zeigt, dass zum Ende der arteriellen Phase der Kammer- und Leitungsdruck auf den gewünschten Rückgabedruck angestiegen sind, wobei der Speicherdruck während der gesamten arteriellen Phase immer unterhalb des Kammer- und Leitungsdrucks, insbesondere unterhalb des Umgebungdrucks liegt. Dadurch ist ausgeschlossen, dass auch bei einem Störfall des Systems, beispielsweise bei einer Leckage des Kompressors, Luft aus dem Luftspeicher in den Blutspeicher gelangt.

Anstelle der Unterteilung der arteriellen Phase in ein erstes und zweites Zeitintervall sind auch andere Ausführungsformen möglich, mit denen sich auch die erfindungsgemäßen Vorteile erzielen lassen. Eine alternative Ausführungsform sieht vor, anstelle des Bypassventils 22 ein mittels der Steuerung druckgeregeltes Ventil einzusetzen, das bei Erreichen eines dem Rückgabedruck entsprechenden Grenzdrucks öffnet, so dass in einem gewissen Zeitbereich ein konstanter Druck im Blutspeicher herrscht.

Anschließend schaltet die Steuer- und Recheneinheit auf die venöse Phase um, wobei das Bypassventil geschlossen bleibt, die Blutpumpe angehalten und der Kompressor in Betrieb gesetzt sowie die venöse Schlauchklemme geöffnet wird. Der Kompressor wird während der gesamten venösen Phase betrieben, wobei die Blutpumpe stillsteht. Während der venösen Phase bleibt die venöse Schlauchklemme geöffnet und das Bypassventil geschlossen.

Der Kompressor fördert Luft aus dem Luftspeicher in den Blutspeicher, um einen Überdruck aufzubauen, so dass Blut aus dem Blutspeicher gefördert wird. Der Kompressor wird dabei derart betrieben, dass sich im Blutspeicher der gewünschte Rückgabedruck einstellt. Da dem Blutspeicher fortwährend Luft aus dem Luftspeicher zugeführt wird, nimmt der Speicherdruck kontinuierlich ab. Entscheidend ist wieder, dass der Speicherdruck immer unterhalb des Kammer- und Leitungsdrucks, vorzugsweise auch unterhalb des Umgebungdrucks liegt, so dass eine Luftinfusion aus dem Luftspeicher in den Patienten bei einem Störfall ausgeschlossen ist. Die venöse Phase ist dann beendet, wenn der Blutpegel im Blutspeicher wieder auf das Niveau des unteren Umschaltpunkts abgesunken ist. Dann schließt sich die nächste arterielle Phase an.

Nachfolgend wird die Berechnung des im Blutspeicher befindlichen Blutvolumens $V_{Blut}$ beschrieben, die von der Steuer- und Recheneinheit während des Betriebs der Dialysevorrichtung kontinuierlich oder in vorgegebenen Intervallen vorgenommen wird. Wenn das Blutvolumen bekannt ist, kann dies zum Vergleich mit den Umschaltpunkten von der arteriellen auf die venöse Phase oder umgekehrt verwendet werden.

Zur Berechnung des in dem Blutspeicher befindlichen Blutvolumens $V_{Blut}$ ist es zunächst notwendig, die Luftmasse im Blutspeicher zu bestimmen. Nach erfolgtem Druckausgleich gilt:

$$pV = \frac{m}{M_m} RT$$

mit:

p = Druck (absolut), V = Volumen, m = Masse, $M_m$ = molare Masse, R = allg. Gaskonstante und T = Temperatur

Da $M_m$ und $R$ bekannt und konstant sind und die absoluten Beträge der Luftmassen als solche nicht benötigt werden, reicht es in der Praxis aus, mit Hilfsgrößen für die Massen zu rechnen, die proportional zur Masse sind, jedoch nicht die Einheit einer Masse haben. Bestimmt werden müssen demnach nur die Hilfsgrößen:

$$\hat{m} = \frac{pV}{T}$$

mit

$$\hat{m} \neq m$$

Zur Bestimmung der Hilfsgröße für die gesamte Luftmasse muss die Summe aller Hilfsgrößen der Teilluftmassen, d.h. der Luftmasse im Blutspeicher, den zugehörigen Leitungen und im Luftspeicher gebildet werden. Hierzu müssen alle mit Luft gefüllten Volumina mit dem jeweils herrschenden Druck multipliziert und durch die Temperatur dividiert werden.

$$\left(\frac{pV}{T}\right)_{ges} = \frac{p_{Blutspeicher} \cdot V_{Blutspeicher / Luft}}{T_{Blutspeicher}} + \frac{p_{Leitung} \cdot V_{Leitung}}{T_{Leitung}} + \frac{p_{Luftspeicher} \cdot V_{Luftspeicher}}{T_{Luftspeicher}}$$

$$T_{Blutspeicher} \approx 273,15 \text{ K} + 36K$$

$$T_{Leitung} \approx \frac{T_{Blutspeicher} + T_{Luftspeicher}}{2}$$

Das Luftvolumen des Blutspeichers $V_{Blutspeicher/Luft}$ ändert sich während des Betriebs. Ist der Blutpegel auf der Höhe H des von dem Füllstandgeber 15 detektierten Niveaus, so entspricht

$$V_{Blutspeicher/Luft} = V_{Blutspeicher/Luft \, UT} - \Delta V_{Blutspeicher/ \, UT-H,}$$

wobei $V_{Blutspeicher/LuftUT}$ das Luftvolumen im Blutspeicher am unteren Umschaltpunkt UT und $\Delta V_{Blutspeicher/ \, UT-H}$ die Volumendifferenz zwischen dem unteren Umschaltpunkt UT und der Höhe des von dem Füllstandgeber 15 detektierten Füllstands im Blutspeicher ist.

Am Ende der Initialisierung am oberen Umschaltpunkt OT befindet sich zusätzlich Blut mit dem gesamten Schlagvolumen $V_{Schlag}$ in dem Blutspeicher, so dass

$$V_{Blutspeicher/Luft} = V_{Blutspeicher/Luft\ UT} - V_{Schlag}$$

ist.

Die restlichen Volumina bleiben konstant. Dabei sind die Temperaturen in guter Näherung als konstant zu setzen, wenn keine Temperaturkompensation erfolgt. Vorzugsweise ist aber ein Temperatursensor zur Temperaturmessung zumindest im Luftspeicher vorgesehen, so dass eine Temperaturkompensation vorgenommen werden kann. Es können aber auch Temperatursensoren für die anderen Druckwerte vorgesehen sein.

Das geförderte Blutvolumen $V_{Blut}$ im Blutspeicher wird während des gesamten Zyklus bei jeder Programmschleife berechnet. Dabei wird anhand der gemessenen Drücke das im Blutspeicher eingeschlossene Luftvolumen $V_{Blutspeicher/Luft}$ berechnet und die Differenz von dem Luftvolumen im Blutspeicher bei dem unteren Umschaltpunkt UT und dem Luftvolumen des Blutspeichers gebildet.

$$V_{Blutspeicher\,/\,Luft} = \frac{\left(\dfrac{pV}{T}\right)_{ges} - \dfrac{P_{Leitung}V_{Leitung}}{T_{Leitung}} - \dfrac{P_{Luftspeicher}V_{Luftspeicher}}{T_{Luftspeicher}}}{\dfrac{P_{Blutspeicher}}{T_{Blutspeicher}}}$$

$$V_{Blut} = V_{Blutspeicher/Luft\ UT} - V_{Blutspeicher/Luft}$$

Die Hilfsgröße für die Gesamtluftmasse $\hat{m}_{ges} = \left(\dfrac{pV}{T}\right)_{ges}$ und die Gesamtmasse $m_{ges}$ bleiben nach der Initialisierung des Systems unverändert, weil das Entlüftungsventil geschlossen bleibt. Da die Berechnung des Blutvolumens $V_{Blut}$ bei laufender Blutpumpe bzw. laufendem Kompressor erfolgt, wird eine Glättung der Drucksignale und des berechneten Blutvolumens $V_{Blut}$ durchgeführt.

Die Umschaltung von arterieller auf venöse Phase (OT) erfolgt, wenn die Differenz von dem berechneten Blutvolumen $V_{Blut}$ und dem eingestellten Schlagvolumen $V_{Schlag}$ gleich Null ist, und die Umschaltung von venöser auf arterielle Phase erfolgt, wenn das berechnete Blutvolumen gleich Null ist.

Im Folgenden wird beschrieben, wie die Steuer- und Recheneinheit den Zeitpunkt berechnet, zu dem innerhalb der arteriellen Phase zwischen dem ersten und zweiten Zeitintervall der arteriellen Phase umgeschaltet wird.

Wie bereits oben beschrieben, ist der Umschaltpunkt von arterieller zu venöser Phase und zurück durch einen einfachen Vergleich des Blutvolumens mit dem Schlagvolumen bzw. Null möglich. Die arterielle Phase teilt sich, wie bereits erwähnt, in ein erstes und ein zweites Zeitintervall auf. In der ersten arteriellen Phase fördert die Blutpumpe Blut über den Dialysator bei geöffnetem Bypassventil in den Blutspeicher. Dadurch wird der Unterdruck, der sich in der venösen Phase zuvor im Luftspeicher aufgebaut hat, zur Unterstützung der Pumpe genutzt. In der zweiten arteriellen Phase wird der Luftspeicher durch Schließen des Bypassventils von dem übrigen System abgekoppelt, und der Druck in dem Blutspeicher steigt durch das geförderte Blutvolumen stark an. Am Ende der zweiten arteriellen Phase soll im Blutspeicher der gewünschte Solldruck anstehen. Der Umschaltpunkt zwischen erster und zweiter arterieller Phase muss daher so gewählt werden, dass das noch ausstehende Blutvolumen bis zum oberen Umschaltpunkt OT in dem Blutspeicher und der Leitung den Solldruck aufbaut.

Berechnet werden muss demnach die Luftmasse, die bei Kompression auf das bei dem oberen Umschaltpunkt OT in dem Blutspeicher und der Leitung verfügbare Luftvolumen den Solldruck $p_{soll}$ aufbaut. Die Luftmasse in Blutspeicher und Leitung ist

$$\frac{p_{Blutspeicher}V_{Blutspeicher}}{T_{Blutspeicher}} + \frac{p_{Leitung}V_{Leitung}}{T_{Leitung}} = \left(\frac{pV}{T}\right)_{ges} - \frac{p_{Luftspeicher}V_{Luftspeicher}}{T_{Luftspeicher}}.$$

Beim Umschaltpunkt von dem ersten auf das zweite Zeitintervall der arteriellen Phase muss die in Blutspeicher und Leitung befindliche Luftmasse gleich der Luftmasse sein, die sich im oberen Umschaltpunkt OT in dem Blutspeicher und der Leitung befindet.

$$\left(\frac{pV}{T}\right)_{ges} - \frac{p_{Luftspeicher}V_{Luftspeicher}}{T_{Luftspeicher}} = \frac{p_{Soll} \cdot V_{Blutspeicher/LuftOT}}{T_{Blutspeicher}} + \frac{p_{Soll} \cdot V_{Leitung}}{T_{Leitung}}$$

Dabei ist $V_{Blutspeicher/LuftOT}$ das Luftvolumen im Blutspeicher am oberen Umschaltpunkt OT.

**Patentansprüche**

1. Vorrichtung zur Blutbehandlung für den Einnadel-Betrieb mit
Mitteln (11) zum Sammeln von Blut, die ein abgeschlossenes Volumen umfassen,
Mitteln (18) zum Speichern von Gas, die ein abgeschlossenes Volumen umfassen,
Mitteln (19, 21) zur Herstellung einer Verbindung zwischen den Mitteln (11) zum Sammeln von Blut und den Mitteln (18) zum Speichern von Gas, wobei die Mittel (11) zum Sammeln von Blut und die Mittel (18) zum Speichern von Gas zusammen <u>mit den Mitteln (19, 21) zur Herstellung einer Verbindung zwischen den Mitteln (11) zum Sammeln von Blut und den Mitteln (18) zum Speichern von Gas</u> ein abgeschlossenes Volumen bilden, <u>in das kein Gas eintreten kann bzw. aus dem kein Gas austreten kann,</u>
Mitteln (6) zum Fördern von Blut in einem extrakorporalen Blutkreislauf zu den Mitteln (11) zum Sammeln von Blut, wobei der extrakorporale Blutkreislauf eine zu einem Einlass (4A) einer Blutbehandlungseinheit (2) führende Blutzuführleitung (8) und eine von einem Auslass (4B) der Blutbehandlungseinheit abgehende Blutrückführleitung (9) aufweist,
Mitteln zum Verdichten (20) von Gas, und
einer Steuer- und Recheneinheit (27), die derart mit den Mitteln (11) zum Sammeln von Blut, mit den Mitteln (18) zum Speichern von Gas, mit den Mitteln (19, 21) zur Herstellung einer Verbindung zwischen den Mitteln (11) zum Sammeln von Blut und den Mitteln (18) zum Speichern von Gas und mit den Mitteln (20) zum Verdichten von Gas zusammenwirkt,
dass in einer arteriellen Phase beim Befüllen der Mittel zum Sammeln von Blut aus den Mitteln zum Sammeln von Blut verdrängtes Gas zu den Mitteln zum Speichern von Gas überführt wird und in einer venösen Phase zum Entleeren der Mittel zum Sammeln von Blut in den Mitteln zum Speichern von Gas gespeichertes Gas mit den Mitteln zum Verdichten von Gas zu den Mitteln zum Sammeln von Blut überführt wird, so dass dem extrakorporalen Blutkreislauf in der arteriellen Phase Blut zugeführt und in der venösen Phase von dem extrakorporalen Blutkreislauf Blut abgeführt werden kann,
**dadurch gekennzeichnet, dass**
die Steuer- und Recheneinheit (27) derart ausgebildet ist, dass
zur Initialisierung der aufeinander folgenden arteriellen und venösen Phasen ein in den Mitteln (18) zum Speichern von Gas einzustellender Druck $p_{LuftspeicherMax}$ berechnet wird,
der zuvor berechnete Druck $p_{LuftspeicherMax}$ vor Einleitung der arteriellen und venösen Phasen in den Mitteln (18) zum Speichern von Gas für die Initialisierung eingestellt wird, und
nach Einstellung des Drucks die arteriellen und venösen Phasen eingeleitet werden.

2. Vorrichtung nach Anspruch 1, **dadurch gekennzeichnet, dass** die Steuer- und Recheneinheit (27) derart ausgebildet ist, dass zum Einstellen des in den Mitteln (18) zum Speichern von Gas einzustellenden Drucks $p_{LuftspeicherMax}$ die Mittel (20) zum Verdichten von Gas betrieben werden, so dass Gas aus den Mitteln (18) zum Speichern von Gas abgeführt oder Gas den Mitteln (18) zum Speichern von Gas zugeführt wird.

3. Vorrichtung nach Anspruch 1 oder 2, **dadurch gekennzeichnet, dass** die Steuer- und Recheneinheit (27) derart ausgebildet ist, dass zur Berechnung des in den Mitteln (18) zum Speichern von Gas einzustellenden Drucks $p_{LuftspeicherMax}$ das in den Mitteln (11) zum Sammeln von Blut enthaltene Volumen an Gas ermittelt wird und auf der Grundlage des in den Mitteln (11) zum Sammeln von Blut enthaltenen Volumens an Gas der in den Mitteln (18) zum Speichern von Gas einzustellende Druck $p_{LuftspeicherMax}$ bestimmt wird.

4. Vorrichtung nach Anspruch 3, **dadurch gekennzeichnet, dass** Mittel (16, 26, 31) zur Bestimmung des absoluten Drucks in den Mitteln (11) zum Sammeln von Blut und den Mitteln (18) zum Speichern von Gas vorgesehen sind, wobei die Steuer- und Recheneinheit (27) derart ausgebildet ist, dass zur Ermittlung des in den Mitteln (11) zum Sammeln von Blut enthaltenen Volumens an Gas zum Aufbau eines vorgegebenen Druckunterschieds zwischen den Mitteln (11) Sammeln von Blut und den Mitteln (18) zum Speichern von Gas Gas aus den Mitteln (18) zum Speichern von Gas in die Mittel (11) zum Sammeln von Blut überführt wird, und der absolute Druck in den Mitteln (18) zum Speichern von Gas und der absolute Druck in den Mitteln (11) zum Sammeln von Blut vor und nach dem Aufbau des Druckunterschieds bestimmt wird.

5. Vorrichtung nach Anspruch 4, **dadurch gekennzeichnet, dass** die Mittel (16, 26, 31) zur Bestimmung des absoluten Drucks Mittel (31) zum Messen des Umgebungsdrucks, Mittel (26) zum Messen des relativen Drucks in den Mitteln (18) zum Speichern von Gas und Mittel (16) zum Messen des relativen Drucks in den Mitteln (11) zum Sammeln von Blut aufweisen, wobei die Steuer- und Recheneinheit (27) derart ausgebildet ist, dass die Werte des absoluten Drucks jeweils aus den gemessenen Werten des relativen Drucks und des Umgebungsdrucks berechnet werden.

6. Vorrichtung nach einem der Ansprüche 1 bis 5, **dadurch gekennzeichnet, dass** die Mittel (19, 21) zur Herstellung einer Verbindung zwischen den Mitteln (11) zum Sammeln von Blut und den Mitteln (18) zum Speichern von Gas einen ersten Verbindungspfad (21) und einen zweiten Verbindungspfad (19) aufweisen, wobei der erste Verbindungspfad eine ein Bypass-Ventil (22) aufweisende Bypass-Leitung (21) aufweist, die die Mittel (11) zum Sammeln von Blut mit den Mitteln (18) zum Speichern von Gas verbindet, und der zweite Verbindungspfad eine Leitung (19) zum Fördern von Gas aufweist, wobei die Mittel (20) zum Verdichten von Gas in der Leitung (19) zum Fördern von Gas angeordnet sind.

7. Vorrichtung nach einem der Ansprüche 4 bis 6, **dadurch gekennzeichnet, dass** die Steuer- und Recheneinheit (27) derart ausgebildet ist, dass vor dem Überführen von Gas aus den Mitteln (18) zum Speichern von Gas in die Mittel (11) zum Sammeln von Blut zum Aufbau eines Druckunterschieds in dem abgeschlossenen Volumen der Mittel (11) zum Sammeln von Blut und dem abgeschlossenen Volumen der Mittel (18) zum Speichern von Gas ein Druck eingestellt wird, der dem Umgebungsdruck entspricht.

8. Vorrichtung nach Anspruch 7, **dadurch gekennzeichnet, dass** Mittel (24) zum Be-/Entlüften vorgesehen sind, wobei die Steuer- und Recheneinheit (27) derart ausgebildet ist, dass zur Einstellung des Umgebungsdrucks eine Verbindung zwischen den Mitteln (18) zum Speichern von Gas und den Mitteln (11) zum Sammeln von Blut hergestellt und die Mittel (18) zum Speichern von Gas und/oder die Mittel (11) zum Sammeln von Blut be-/entlüftet werden.

9. Vorrichtung nach Anspruch 6, **dadurch gekennzeichnet, dass** die Leitung zum Fördern von Gas (19) einen ersten Leitungsabschnitt (19A) aufweist, der die Mittel (11) zum Sammeln von Blut mit der Druckseite der Mittel (20) zum Verdichten von Gas verbindet, und einen zweiten Leitungsabschnitt (19B) aufweist, der die Saugseite der Mittel zum Verdichten von Gas mit den Mitteln zum Speichern (18) von Gas verbindet, wobei die Mittel (24B) zum Be- / Entlüften in dem ersten Leitungsabschnitt vorgesehen sind.

10. Blutbehandlungsvorrichtung nach einem der Ansprüche 1 bis 9, **dadurch gekennzeichnet, dass** die Mittel (11) zum Sammeln von Blut als Behälter mit einem vorgegebenen Volumen ausgebildet sind, der in der Blutrückführleitung (9) des extrakorporalen Kreislaufs (1) angeordnet ist.

11. Blutbehandlungsvorrichtung nach einem der Ansprüche 1 bis 10, **dadurch gekennzeichnet, dass** die Mittel (18) zum Speichern von Gas als ein Behälter mit einem vorgegebenen Volumen ausgebildet sind.

12. Blutbehandlungsvorrichtung nach einem der Ansprüche 1 bis 10, **dadurch gekennzeichnet, dass** die Mittel (6) zum Fördern von Blut im extrakorporalen Kreislauf (1) als Blutpumpe ausgebildet sind, die in der Blutzuführleitung (8) angeordnet ist.

13. Blutbehandlungsvorrichtung nach einem der Ansprüche 1 bis 12, **dadurch gekennzeichnet, dass** die Steuer- und Recheneinheit Mittel (27) zum Umschalten zwischen der arteriellen Phase und der venösen Phase aufweist, die derart ausgebildet sind,
dass in der arteriellen Phase die Mittel (6) zum Fördern von Blut im extrakorporalen Kreislauf (1) betrieben werden, wobei die Mittel (11) zum Sammeln von Blut mit den Mitteln (18) zum Speichern von Gas in Verbindung stehen, so dass beim Befüllen der Mittel zum Sammeln von Blut aus den Mitteln zum Sammeln von Blut verdrängtes Gas zu den Mitteln zum Speichern von Gas überführt wird, und
dass in der venösen Phase die Mittel (20) zum Verdichten von Gas betrieben werden, wobei die Mittel (18) zum Speichern von Gas mit den Mitteln (11) zum Sammeln von Blut in Verbindung stehen, so dass zum Entleeren der Mittel zum Sammeln von Blut in den Mitteln zum Speichern von Gas gespeichertes Gas mit den Mitteln zum Verdichten von Gas zu den Mitteln zum Sammeln von Blut überführt wird.

**Claims**

1. Apparatus for blood treatment for single-needle operation, comprising:

   blood collection means (11) which comprise a sealed volume;
   gas storage means (18) which comprise a sealed volume;
   means (19, 21) for making a connection between the blood collection means (11) and the gas storage means (18), the blood collection means (11) and the gas storage means (18) forming, together with the means (19, 21) for making a connection between the blood collection means (11) and the gas storage means (18), a sealed volume, which no gas can enter and from which no gas can exit;
   means (6) for feeding blood to the blood collection means (11) in an extracorporeal blood circuit, the extracorporeal blood circuit having a blood infeed line (8) which runs to an inlet (4A) of a blood treatment unit (2) and a blood return line (9) which leads away from an outlet (4B) of the blood treatment unit;
   gas compression means (20); and
   a control and arithmetic unit (27) which co-operates with the blood collection means (11), the gas storage means (18), the means (19, 21) for making a connection between the blood collection means (11) and the gas storage means (18), and the gas compression means (20) such that,
   in an arterial phase when the blood collection means is being filled, gas which is displaced from the blood collection means is transferred to the gas storage means and, in a venous phase for emptying the blood collection means, gas which has been stored in the gas storage means is transferred to the blood collection means by the gas compression means such that blood can be fed to the extracorporeal blood circuit in the arterial phase and discharged from the extracorporeal blood circuit in the venous phase,
   **characterised in that**
   the control and arithmetic unit (27) is configured such that,
   to initialise the successive arterial and venous phases, a pressure Pair reservoir max which is to be set in the gas storage means (18) is calculated,
   for the initialisation, the previously calculated pressure $p_{air\ reservoir\ max}$ is set in the gas storage means (18) before the arterial and venous phases are started and, once the pressure is set, the arterial and venous phases are started.

2. Apparatus according to claim 1, **characterised in that** the control and arithmetic unit (27) is configured such that, to set the pressure $p_{air\ reservoir\ max}$ which is to be set in the gas storage means (18), the gas compression means (20) is operated such that gas is discharged from the gas storage means (18) or gas is fed to the gas storage means (18).

3. Apparatus according to either claim 1 or claim 2, **characterised in that** the control and arithmetic unit (27) is configured such that, to calculate the pressure $P_{air\ reservoir\ max}$ which is to be set in the gas storage means (18), the volume of gas contained in the blood collection means (11) is determined and the pressure $P_{air}$ reservoir max which is to be set in the gas storage means (18) is determined on the basis of the volume of gas contained in the blood collection means (11).

4. Apparatus according to claim 3, **characterised in that** means (16, 26, 31) for determining the absolute pressure are provided in the blood collection means (11) and the gas storage means (18), the control and arithmetic unit (27) being configured such that, to determine the volume of gas contained in the blood collection means (11), gas is transferred from the gas storage means (18) to the blood collection means (11) to produce a preset difference in pressure between the blood collection means (11) and the gas storage means (18), and the absolute pressure in the gas storage means (18) and the absolute pressure in the blood collection means (11) are determined before and after production of the difference in pressure.

5. Apparatus according to claim 4, **characterised in that** the means (16, 26, 31) for determining the absolute pressure include means (31) for measuring the ambient pressure, means (26) for measuring the relative pressure in the gas storage means (18) and means (16) for measuring the relative pressure in the blood collection means (11), the control and arithmetic unit (27) being configured such that the values of the absolute pressure are calculated in each case from the measured values of the relative pressure and the ambient pressure.

6. Apparatus according to any of claims 1 to 5, **characterised in that** the means (19, 21) for making a connection between the blood collection means (11) and the gas storage means (18) have a first connecting path (21) and a second connecting path (19), the first connecting path having a bypass line (21) which has a bypass valve (22) and connects the blood collection means (11) to the gas storage means (18), and the second connecting path having a gas conveying line (19), the gas compression means (20) being arranged in the gas conveying line (19).

7. Apparatus according to any of claims 4 to 6, **characterised in that** the control and arithmetic unit (27) is configured such that, before gas is transferred from the gas storage means (18) to the blood collection means (11), a pressure which corresponds to the ambient pressure is set to produce a difference in pressure in the sealed volume in the blood collection means (11) and in the sealed volume in the gas storage means (18).

8. Apparatus according to claim 7, **characterised in that** means (24) for air admission/release are provided, the control and arithmetic unit (27) being configured such that, to set the ambient pressure, a connection is made between the gas storage means (18) and the blood collection means (11), and air is admitted to/released from the gas storage means (18) and/or the blood collection means (11).

9. Apparatus according to claim 6, **characterised in that** the gas conveying line (19) has a first line portion (19A) which connects the blood collection means (11) to the discharge side of the gas compression means (20), and a second line portion (19B) which connects the inlet side of the gas compression means to the gas storage means (18), the means (24B) for air admission/release being provided in the first line portion.

10. Blood treatment apparatus according to any of claims 1 to 9, **characterised in that** the blood collection means (11) takes the form of a container which has a preset volume and is arranged in the blood return line (9) of the extracorporeal blood circuit (1).

11. Blood treatment apparatus according to any of claims 1 to 10, **characterised in that** the gas storage means (18) takes the form of a container having a preset volume.

12. Blood treatment apparatus according to any of claims 1 to 10, **characterised in that** the means (6) for conveying blood in the extracorporeal blood circuit (1) takes the form of a blood pump which is arranged in the blood infeed line (8).

13. Blood treatment apparatus according to any of claims 1 to 12, **characterised in that** the control and arithmetic unit has means (27) for switching between the arterial phase and the venous phase, which means are configured such that the means (6) for feeding blood in the extracorporeal blood circuit (1) are operated in the arterial phase, the blood collection means (11) being connected to the gas storage means (18) such that gas displaced from the blood collection means is transferred to the gas storage means when the blood collection means is being filled, and the gas compression means (20) are operated in the venous phase, the gas storage means (18) being connected to the blood collection means (11) such that gas which has been stored in the gas storage means is transferred to the blood collection means by the gas compression means in order to empty the blood collection means.

**Revendications**

1. Dispositif de traitement du sang à aiguille unique, comportant
   des moyens (11) de collecte de sang qui comprennent un volume fermé,
   des moyens (18) d'emmagasinement de gaz, qui comprennent un volume fermé,
   des moyens (19, 21) de production d'une liaison entre les moyens (11) de collecte de sang et les moyens (18) d'emmagasinement de gaz, dans lequel les moyens (11) de collecte de sang et les moyens (18) d'emmagasinement de gaz conjointement avec les moyens (19, 21) pour la production d'une liaison entre les moyens (11) de collecte de sang et les moyens (18) d'emmagasinement de gaz forment un volume fermé dans lequel aucun gaz ne peut pénétrer ni sortir,
   des moyens (6) d'acheminement de sang dans une circulation sanguine extracorporelle aux moyens (11) de collecte de sang, dans lequel la circulation extracorporelle du sang présente une conduite d'apport de sang (8) conduisant à l'entrée (4A) d'une unité de traitement de sang (2) et une conduite de retour de sang (9) partant d'une sortie (4B) de l'unité de traitement de sang,
   des moyens de compression (20) de gaz, et
   une unité de commande et de calcul (27) qui coopère avec les moyens (11) de collecte de sang, avec les moyens (18) d'emmagasinement de gaz, avec les moyens (19, 21) de production d'une liaison entre les moyens (11) de collecte de sang et les moyens (18) d'emmagasinement de gaz et avec les moyens (20) de compression de gaz, de telle sorte que dans une phase artérielle, lors du remplissage des moyens de collecte de sang à partir des moyens de collecte de sang, du gaz déplacé est transféré vers les moyens d'emmagasinement de gaz et dans une phase veineuse pour vider les moyens de collecte de sang dans les moyens d'emmagasinement de gaz, du gaz emmagasiné est transféré avec les moyens de compression de gaz aux moyens de collecte de sang de sorte que du sang soit conduit à la circulation extracorporelle du sang dans la phase artérielle et dans la phase veineuse, du sang peut être évacué de la circulation extracorporelle du sang,

**caractérisé en ce que**

l'unité de commande et de calcul (27) est conçue de telle sorte que

pour l'initialisation des phases artérielles et veineuses consécutives, une pression $P_{emmag. \, d'air \, max}$ à régler dans les moyens (18) d'emmagasinement de gaz est calculée,

la pression $P_{emmag. \, d'air \, max}$ calculée précédemment est réglée avant l'introduction des phases artérielles et veineuses dans les moyens (18) d'emmagasinement de gaz pour l'initialisation, et

après le réglage de la pression, les phases artérielles et veineuses sont introduites.

2. Dispositif selon la revendication 1, **caractérisé en ce que** l'unité de commande et de calcul (27) est conçue de telle sorte que pour le réglage de la pression $P_{emmag. \, d'air \, max}$ à régler dans les moyens (18) d'emmagasinement de gaz, les moyens (20) de compression de gaz sont actionnés de sorte que du gaz soit évacué des moyens (18) d'emmagasinement de gaz ou que du gaz soit apporté aux moyens (18) d'emmagasinement de gaz.

3. Dispositif selon la revendication 1 ou 2, **caractérisé en ce que** l'unité de commande et de calcul (27) est conçue de telle sorte que pour le calcul de la pression $P_{emmag. \, d'air \, max}$ à régler dans les moyens (18) d'emmagasinement de gaz, le volume de gaz contenu dans les moyens (11) de collecte de sang est déterminé et sur la base du volume de gaz contenu dans les moyens (11) de collecte de sang, la pression $P_{emmag. \, d'air \, max}$ à régler dans les moyens (18) d'emmagasinement de gaz, est déterminée.

4. Dispositif selon la revendication 3, **caractérisé en ce que** des moyens (16, 26, 31) de détermination de la pression absolue sont prévus dans les moyens (11) de collecte de sang et les moyens (18) d'emmagasinement de gaz, dans lequel l'unité de commande et de calcul (27) est conçue de telle sorte que pour la détermination du volume de gaz contenu dans les moyens (11) de collecte de sang, pour la constitution d'une différence de pression prédéterminée entre les moyens (11) de collecte de sang et les moyens (18) d'emmagasinement de gaz, du gaz est transféré des moyens (18) d'emmagasinement de gaz dans les moyens (11) de collecte de sang et la pression absolue dans les moyens (18) d'emmagasinement de gaz et la pression absolue dans les moyens (11) de collecte de sang est déterminée avant et après la constitution de la différence de pression.

5. Dispositif selon la revendication 4, **caractérisé en ce que** les moyens (16, 26, 31) de détermination de la pression absolue présentent des moyens (31) de mesure de la pression ambiante, des moyens (26) de mesure de la pression relative dans les moyens (18) d'emmagasinement de gaz et des moyens (16) de mesure de la pression relative dans les moyens (11) de collecte de sang, dans lequel l'unité de commande et de calcul (27) est conçue de telle sorte que les valeurs de la pression absolue sont calculées respectivement à partir des valeurs mesurées de la pression relative et de la pression ambiante.

6. Dispositif selon l'une des revendications 1 à 5, **caractérisé en ce que** les moyens (19, 21) de production d'une liaison entre les moyens (11) de collecte de sang et les moyens (18) d'emmagasinement de gaz présentent un premier chemin de liaison (21) et un deuxième chemin de liaison (19), dans lequel le premier chemin de liaison présente une conduite de dérivation (21) présentant une valve de dérivation (22) qui relie les moyens (11) de collecte de sang aux moyens (18) d'emmagasinement de gaz et le deuxième chemin de liaison présente une conduite (19) pour l'acheminement de gaz, dans lequel les moyens (20) de compression de gaz sont disposés dans la conduite (19) pour l'acheminement de gaz.

7. Dispositif selon l'une des revendications 4 à 6, **caractérisé en ce que** l'unité de commande et de calcul (27) est conçue de telle sorte qu'avant le transfert du gaz des moyens (18) d'emmagasinement de gaz dans les moyens (11) de collecte de sang pour la constitution d'une différence de pression dans le volume fermé des moyens (11) de collecte de sang et dans le volume fermé des moyens (18) d'emmagasinement de gaz, une pression est réglée, qui correspond à la pression ambiante.

8. Dispositif selon la revendication 7, **caractérisé en ce que** des moyens (24) de ventilation/aération sont prévus, dans lequel l'unité de commande et de calcul (27) est conçue de telle sorte que le réglage de la pression ambiante établit une liaison entre les moyens (18) d'emmagasinement de gaz et les moyens (11) de collecte de sang et les moyens (18) d'emmagasinement de gaz et/ou les moyens (11) de collecte de sang sont ventilés/aérés.

9. Dispositif selon la revendication 6, **caractérisé en ce que** la conduite d'acheminement de gaz (19) présente un premier segment de conduite (19A) qui relie les moyens (11) de collecte de sang avec le côté de compression des moyens (20) de compression de gaz et un deuxième segment de conduite (19B) qui relie le côté d'aspiration des moyens de compression de gaz avec les moyens (18) d'emmagasinement de gaz, dans lequel des moyens (24B) de ventilation/aération sont prévus dans le premier segment de conduite.

10. Dispositif de traitement de sang selon l'une des revendications 1 à 9, **caractérisé en ce que** les moyens (11) de collecte de sang sont constitués comme des conteneurs présentant un volume prédéterminé qui sont montés dans la conduite de retour de sang (9) de la circulation extracorporelle (1).

11. Dispositif de traitement de sang selon l'une des revendications 1 à 10, **caractérisé en ce que** les moyens (18) d'emmagasinement de gaz sont conçus comme un conteneur présentant un volume prédéterminé.

12. Dispositif de traitement de sang selon l'une des revendications 1 à 10, **caractérisé en ce que** les moyens (6) d'acheminement de sang dans la circulation extracorporelle (1) sont conçus comme une pompe de sang qui est montée dans la conduite d'apport de sang (8).

13. Dispositif de traitement de sang selon l'une des revendications 1 à 12, **caractérisé en ce que** l'unité de commande et de calcul présente des moyens (27) qui sont conçus pour commuter entre la phase artérielle et la phase veineuse de telle sorte

que dans la phase artérielle, les moyens (6) d'acheminement de sang dans la circulation extracorporelle sont actionnés, dans lequel les moyens (11) de collecte de sang sont en liaison avec les moyens (18) d'emmagasinement de gaz de sorte que lors du remplissage des moyens de collecte de sang à partir des moyens de collecte de sang, du gaz déplacé est transféré vers les moyens d'emmagasinement de gaz, et

que dans la phase veineuse les moyens (20) de compression de gaz sont actionnés, dans lequel les moyens (18) d'emmagasinement de gaz sont en liaison avec les moyens (11) de collecte de sang de sorte que pour le vidage des moyens de collecte de sang dans les moyens d'emmagasinement de gaz du gaz emmagasiné est transféré avec les moyens de compression de gaz aux moyens de collecte de sang.

Fig. 1

EP 2 575 924 B1

Fig. 2

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0405094 A2
- DE 102007026009 A1
- DE 102009024468
- EP 2010002488 W